# EUROPEAN PATENT APPLICATION

(11) **EP 1 163 852 A1**
(43) Date of publication of application: **19.12.2001**
(21) Application number: 00902894.5
(22) Date of filing: 09.02.2000
(51) Int. Cl.: A23L 1/015

(54) **MASKING AGENT**

(30) Priority: 18.02.1999 JP 4031299
(71) Applicant: FUJISAWA PHARMACEUTICAL CO., LTD., Osaka-shi Osaka 541-8514 (JP)
(72) Inventor: NAKAMURA, Tomoyasu, Chiba-shi, Chiba 262-0026 (JP); OGUNI, Nobutaka, Kobe-shi, Hyogo 654-0111 (JP)
(74) Representative: Gille Hrabal Struck Neidlein Prop Roos
(86) International application number: JP0000733
(87) International publication number: WO0048475

(57) **Abstract**

An unpleasant taste and/or smell of a substance to be orally administered are masked by adding thereto a masking agent containing a nontoxic gluconic acid salt as the active ingredient.

## Description

### Technical field

The present invention relates to a masking agent comprising a nontoxic salt of gluconic acid as an active ingredient, an oral ingestible product added with the masking agent, and a method of masking flavor or odor of an oral ingestible product by adding the masking agent.

### Background Art

Tastes for oral ingestible product such as food and drink differ from person to person. In general, the tastes therefor tend to vary depending on various factors such as flavor and odor. Accordingly, even if food is excellent in nutritive value or advantageous in health maintenance/enhancement, it is sometimes disliked because of its unpleasant flavor and odor, and thus difficult to use.

For example, bitter gourd is effective in inhibiting sugar absorption and hot pepper is effective in promoting fat metabolism with its pungent component (capsaicin). Further, soya milk is a healthy drink which is a convenient supply source of soybean protein. However, they are often disliked because of bitterness, pungency and particular odor, respectively.

Polyunsaturated fatty acids contained in fish such as docosahexaenoic acid (DHA), eicosapentaenoic acid (EPA) and the like are recognized as having functions good for health. However, they have a fishy smell and cannot easily be ingested in their natural forms. Accordingly, there has been attempted to reduce the fishy smell by emulsifying or coating.

Further, carotene-rich vegetables are noticed as an important supply source of vitamins and minerals but often disliked because of their raw smell. Garlic having a strong smell and old rice having a particular smell due to oxidation or the like during a storage period are also disliked.

According to development in wrapping materials and processing techniques, various food forms have been provided. A retort-pouched food, which is a typical example thereof, has a problem of a particular smell called a retort smell.

Since animal meats emit a strong smell particular to them, processed food products containing a large amount of them as a material tend to be disliked. Among the animal meats, mutton is inexpensive and easily used as a material for processed livestock products. Accordingly, its utility value increases if the smell of the mutton can be masked.

Powdered skim milk and WPC (Whey Protein Concentrate) are noticed as low fat products as compared with milk so that they are widely utilized as materials for livestock products, marine products, soup, desserts and the like. However, they have powdery flavor and smell particular to them, so that an addition amount thereof to food is limited.

On the other hand, sodium gluconate is known as an additive to a coagulating agent for Tofu. Calcium gluconate is widely used as a calcium supplement. Further, sodium gluconate is known as being effective in improving flavor of high potency sweetener (aspartame, α -L-aspartyl-L-phenylalanine methylester) (International Patent Application No. WO94/09650), but not recognized as an agent for masking bitterness or odor.

It has been reported that polyphenol or trehalose is effective in masking the odor. However, the effect is not satisfactory. Some organic acid salts such as sodium malate, sodium lactate, sodium citrate and the like are recognized as reducing the odor, but the organic acid salts themselves have strong flavors, respectively, so that they cannot be used for masking the odor.

Therefore, there has been expected development of a strong masking agent capable of reducing unpleasant flavor or odor.

### Disclosure of the Invention

The inventors of the present invention have found a masking agent containing a nontoxic salt of gluconic acid as an active ingredient, an oral ingestible product added with the masking agent, and a method of masking flavor or odor of the oral ingestible product by adding the masking agent.

According to the invention, the oral ingestible product is an object to be ingested through the mouth of a human being. Typical examples thereof include foods, drinks, favorite foods, food additives, drugs and the like. The masking agent of the present invention masks odor or flavor, or both of them that are not suitable for a human taste.

Examples of the nontoxic salts of gluconic acid include alkali metal salts of gluconic acid such as sodium gluconate, potassium gluconate and the like, and alkali earth metal salts of gluconic acid such as calcium gluconate, magnesium gluconate and the like. Among them, sodium gluconate and calcium gluconate are particularly preferable.

The masking agent of the present invention may be formulated in the form of powder, granule, tablet, solution and the like solely from the gluconic acid salt or by appropriately mixing the gluconic acid salt with various additives or media according to a conventional method. The content of the gluconic acid salt in these formulations is optionally determined.

Examples of the additives used for formulating the masking agent in the form of powder, granule or tablet include: dietary fibers such as apple fiber, corn fiber, alginic acid, carrot powder, pectin, seaweed polysaccharide, carboxymethyl cellulose and the like; excipients such as lactose, starch and the like; sweeteners such as saccharose, maltose, fructose, sorbitol, mannitol, stevioside, aspartame and the like; nutrition supplements such as vitamin, mineral, milk powder, meat extract and the like; flavoring agents; binders such as powdered acacia, polyvinyl pyrrolidone, hydroxypropyl cellulose and the like; and lubricants such as magnesium stearate, calcium stearate, talc and the like. One or plural kinds of them may appropriately be selected for use.

The solution formulation can be obtained in general by dissolving or suspending the gluconic acid salt in a solvent capable of dissolving it. Examples thereof include water, alcohols such as ethanol, propylene glycol and the like.

A method of adding the gluconic acid salt to the oral ingestible product to be masked is not particularly limited. It may be carried out optionally by mixing, sprinkling or spraying the gluconic acid salt to the object or a material thereof during processing, cooking or ingesting the object.

An addition amount of the masking agent may suitably be increased or decreased depending on the type and strength of the flavor and odor of the object. An effective addition amount can be determined suitably by table tests, for example. In general, the addition amount of the masking agent may be 0.1 to 10 wt%, preferably 0.1 to 5.0 wt% with respect to the object whose flavor or odor should be masked. For example, as shown in Examples to be mentioned later, the addition amount with which sufficient effect will be obtained may be 0.5 to 2.5 wt% with respect to a 30% bitter gourd solution, 0.5 to 1.9 wt% with respect to soya milk, 0.1 to 0.4 wt% with respect to a 20-fold dilution of fish sauce, 1.0 to 5.0 wt% with respect to an emulsion containing 23% DHA, 0.5 to 3.0 wt% with respect to grated garlic, and regarding old rice, 0.1 to 2.0 wt% with respect to water added to cook the old rice.

The masking agent according to the present invention is applicable to the masking of flavor and/or odor of various oral ingestible product. The flavor signifies bitterness, astringency, pungency, sourness and the like. The odor signifies odors of soybean, fish, vegetable, old rice, konjak jelly, vitamin, retort-pouched food, animal meat, powdered milk and the like. Examples of the oral ingestible product to which the masking agent of the invention is applicable include: foods such as bitter gourd, grated Japanese radish, vinegar, pickled Ume, lemon, soya milk, soybean protein, fish sauce, dried bonito shavings, vegetable juice, tomato juice, garlic, old rice, konjak jelly, animal meats (e.g., mutton, beef, beef organ meat, pork, pork organ meat and chicken); favorite foods such as horseradish, hot pepper, beer and the like; food additives such as magnesium chloride, DHA and the like; nutrients or health foods such as propolis, agaricus and Chlorella; vitamins such as vitamin B group; food additives such as powdered skim milk and WPC; and so-called retort-pouched foods. The flavor and odor and the oral ingestible product having such flavor and odor are not limited to those described above.

### Best Mode for Carrying Out the Invention

In the following examples, explained are various oral ingestible product added with the gluconic acid salt and evaluation of an effect of the gluconic acid salt on reduction of flavor or odor thereof. However, the present invention is not limited thereto.

The evaluation of the masking of the flavor or odor was carried out in accordance with the following manner unless particularly mentioned.

Specimens prepared by adding the masking agent were subjected to an organoleptic test by 10 examinees under the evaluation basis described below. Results of the evaluation are expressed as an average of values obtained from all the examinees. The addition amount of the masking agent is indicated by wt%. Evaluation basis
0: particular flavor or odor that should be masked is not perceived at all
1: particular flavor or odor that should be masked is slightly perceived
2: particular flavor or odor that should be masked is perceived
3: particular flavor or odor that should be masked is considerably perceived

### Example 1 Reduction of bitterness

### (1) Bitter gourd

Specimens were prepared by adding 0 to 3 % of sodium gluconate (GNA) to bitter gourd drink commercialized by Okinawa Pokka Corporation (containing 30 % bitter gourd juice, ingredients: bitter gourd juice, high fructose corn syrup, lemon juice, honey, acidifier, coloring agents (safflower yellow, gardenia), stabilizer (pectin), flavoring agent, vitamin C). The specimens were subjected to an organoleptic test to evaluate reduction of bitterness.

**Table 1**

| | | | | | | | |
|---|---|---|---|---|---|---|---|
| Addition amount of GNA (%) | 0 | 0.5 | 1.0 | 1.5 | 2.0 | 2.5 | 3.0 |
| Evaluation results | 3 | 2.5 | 2.1 | 0.8 | 0.6 | 0.2 | 0 |

As shown in Table 1, the addition of GNA to the bitter gourd drink remarkably reduced the bitterness particular to the bitter gourd. The bitterness was not perceived at all when 3% of GNA was added.

### (2) Propolis

2 mL of a propolis solution, comercialized by Mannan Foods Co., Ltd. (originally from China), was mixed with distilled 400 mL of water to prepare samples. To the samples, 0 to 3 % of GNA was added to obtain specimens. The specimens were subjected to the organoleptic test to evaluate reduction of bitterness.

**Table 2**

| | | | | | |
|---|---|---|---|---|---|
| Addition amount of GNA (%) | 0 | 0.5 | 1.0 | 2.0 | 3.0 |
| Evaluation results | 3 | 2.6 | 2.1 | 1.8 | 1.1 |

As shown in Table 2, the bitterness particular to the propolis was reduced by the addition of GNA.

### (3) Magnesium

To a solution of 1 wt% magnesium chloride (MgCl₂·6H₂O) in distilled water prepared as a sample, 0 to 3 % of GNA was added to prepare specimens. The specimens were subjected to the organoleptic test to evaluate reduction of bitterness.

**Table 3**

| | | | | | | |
|---|---|---|---|---|---|---|
| Addition amount of GNA (%) | 0 | 0.5 | 1.0 | 1.5 | 2.0 | 3.0 |
| Evaluation results | 3 | 1.9 | 1.0 | 0.6 | 0.4 | 0 |

As shown in Table 3, the addition of GNA remarkably reduced the bitterness particular to the magnesium chloride. Where 1.5 % or more of GNA was added, the specimens showed a salty taste.

### (4) Beer

To Lager Beer manufactured by Kirin Brewery Co., Ltd. as a sample, 0 to 1% of GNA was added to prepare specimens. The specimens were subjected to the organoleptic test to evaluate reduction of bitterness.

**Table 4**

| | | | | | |
|---|---|---|---|---|---|
| Addition amount of GNA (%) | 0 | 0.1 | 0.3 | 0.5 | 1.0 |
| Evaluation results | 3 | 2.6 | 1.7 | 1.4 | 1.4 |

As shown in Table 4, the bitterness of the beer was reduced by adding GNA.

### Example 2 Reduction of astringency

### (5) Iron

To a 100 mL solution of 0.28 g iron (II) chloride (FeCl₃ 6H₂O) in distilled water (1000 mL) containing 2 mg of Fe prepared as a sample, 0 to 3 % of GNA was added to prepare specimens. The specimens were subjected to the organoleptic test to evaluate reduction of astringency particular to the iron.

**Table 5**

| | | | | | |
|---|---|---|---|---|---|
| Addition amount of GNA (%) | 0 | 0.5 | 1.0 | 2.0 | 3.0 |
| Evaluation results | 3 | 1.8 | 1.4 | 0.9 | 0.6 |

As shown in Table 5, the astringency particular to the iron was reduced by adding GNA.

### Example 3 Reduction of pungency

### (6) Horseradish

2 g of grated horseradish manufactured by S&B Foods Inc. was dissolved in 100mL of distilled water to prepare samples, and then 0 to 3 % of GNA was added thereto to obtain specimens. The specimens were subjected to the organoleptic test to evaluate reduction of pungency.

**Table 6**

| | | | | | |
|---|---|---|---|---|---|
| Addition amount of GNA (%) | 0 | 0.5 | 1.0 | 2.0 | 3.0 |
| Evaluation results | 3 | 2.2 | 1.9 | 1.7 | 1.2 |

As shown in Table 6, the pungency of the horseradish was reduced by adding GNA.

### (7) Hot pepper pickled in Awamori

A liquid portion of 2 mL taken from hot pepper pickled in Awamori made in Okinawa was diluted with 100 mL of distilled water to prepare samples. To the samples, 0 to 3 % of GNA was added to obtain specimens. The specimens were subjected to the organoleptic test to evaluate reduction of pungency.

**Table 7**

| | | | | | |
|---|---|---|---|---|---|
| Addition amount of GNA (%) | 0 | 0.5 | 1.0 | 2.0 | 3.0 |
| Evaluation results | 3 | 2.5 | 2.2 | 1.8 | 1.3 |

As shown in Table 7, the pungency derived from the hot pepper was reduced by adding GNA.

### (8) Kimchee seasoning

Kimchee seasoning, manufactured by Momoya Co., Ltd., was 30-fold diluted with distilled water to prepare samples and 0 to 5 % of GNA was added thereto to obtain specimens. The specimens were subjected to the organoleptic test to evaluate reduction of pungency of hot pepper.

**Table 8**

| | | | | | | |
|---|---|---|---|---|---|---|
| Addition amount of GNA (%) | 0 | 0.5 | 1.0 | 2.0 | 3.0 | 5.0 |
| Evaluation results | 3 | 2.3 | 1.8 | 1.5 | 1.2 | 0.9 |

As shown in Table 8, the pungency of the hot pepper was reduced by adding GNA.

### (9) Grated Japanese radish

To a sample of grated Japanese radish, 0 to 5 % of GNA was added to prepare specimens. The specimens were subjected to the organoleptic test to evaluate reduction of pungency of the Japanese radish.

**Table 9**

| | | | | | | |
|---|---|---|---|---|---|---|
| Addition amount of GNA (%) | 0 | 0.5 | 1.0 | 2.0 | 3.0 | 5.0 |
| Evaluation results | 3 | 2.2 | 2.0 | 1.6 | 1.5 | 0.8 |

As shown in Table 9, the pungency of the Japanese radish was reduced by the addition of GNA.

### Example 4 Reduction of sourness

### (10) Vinegar

8 mL (acidity 4.2 %) of brewed vinegar was diluted with 100 mL of distilled water to prepare samples and 0 to 3 % of GNA was added thereto to obtain specimens. The specimens were subjected to the organoleptic test to evaluate reduction of sourness.

**Table 10**

| | | | | | |
|---|---|---|---|---|---|
| Addition amount of GNA (%) | 0 | 0.5 | 1.0 | 2.0 | 3.0 |
| Evaluation results | 3 | 1.9 | 1.8 | 1.3 | 0.8 |

As shown in Table 10, the sourness of the vinegar was reduced by adding GNA.

### (11) Pickled Ume

A 10 g paste of commercially available pickled Ume was diluted with 100 mL of distilled water to prepare samples and 0 to 3 % of GNA was added thereto to obtain specimens. The specimens were subjected to the organoleptic test to evaluate reduction of sourness of the pickled Ume.

**Table 11**

| | | | | | |
|---|---|---|---|---|---|
| Addition amount of GNA (%) | 0 | 0.5 | 1.0 | 2.0 | 3.0 |
| Evaluation results | 3 | 2.8 | 2.7 | 1.9 | 1.5 |

As shown in Table 11, the sourness of the pickled Ume was reduced by the addition of GNA.

### (12) Lemon

30 mL of lemon juice was diluted with distilled water of 100 mL to prepare samples and 0 to 3 % of GNA was added thereto to obtain specimens. The specimens were subjected to the organoleptic test to evaluate reduction of sourness of lemon.

**Table 12**

| | | | | | |
|---|---|---|---|---|---|
| Addition amount of GNA (%) | 0 | 0.5 | 1.0 | 2.0 | 3.0 |
| Evaluation results | 3 | 2.3 | 1.8 | 1.4 | 0.8 |

As shown in Table 12, the sourness of the lemon was reduced by the addition of GNA.

### Example 5 Reduction of soybean smell

### (13) Soya milk

To soya milk ("Tofu no dekiru tonyu" manufactured by Nagoya Seiraku Co., Ltd.), sodium gluconate, potassium gluconate and calcium gluconate were added, respectively, to obtain specimens. The specimens were subjected to an organoleptic test carried out by 20 examinees. The test was carried out by a paired preference test in which the examinees tasted 2 items once a day. Temperature of the specimens during the test was 20°C. The evaluation by the organoleptic test was carried out as follows.

A threshold value was established by an addition amount with which 18 or more examinees perceived no soybean smell after drinking a first pair of soya milk added with 0.1 % GNA and that added with 0.2 % GNA, and then a second pair of soya milk added with 0.2 % GNA and that added with 0.3 % GNA (significance level 0.1 %). A specimen of soya milk added with 2.5 % sugar and GNA was also prepared and the threshold value was obtained in the same manner as the above.

**Table 13**

| Specimens | | Threshold value |
|---|---|---|
| Soya milk | Sodium gluconate | 0.9 % |
| Soya milk | Potassium gluconate | 1.3 % |
| Soya milk | Calcium gluconate | 0.5 % |
| Soya milk added with 2.5% sugar | Sodium gluconate | 1.6 % |

The results of the test show that the soybean smell particular to the soya milk was reduced by adding 0.5 % or more of the gluconic acid salts. Regarding the soya milk added with sugar, the soybean smell was difficult to perceive because of sweetness of the sugar, but it was confirmed that the soybean smell was killed by adding 1.6 % or more of GNA.

### (14) Vienna sausage containing soybean protein

To a Vienna sausage (containing 5.0 wt% of soybean protein "Sanraba-10" manufactured by Fuji Oil Co., Ltd.), 0 to 5 % of GNA was added to prepare specimens. The specimens were subjected to the organoleptic test to evaluate reduction of the soybean smell.

**Table 14**

| | | | | | | |
|---|---|---|---|---|---|---|
| Addition amount of GNA (%) | 0 | 1 | 2 | 3 | 4 | 5 |
| Evaluation results | 3 | 1.6 | 1.3 | 1.2 | 0.7 | 0.4 |

As shown in Table 14, the addition of GNA to the Vienna sausage containing the soybean protein of 5 wt% reduced the soybean smell derived from the soybean protein.

### (15) Hamburger steak containing soybean protein

To a hamburger steak (containing 5.0 wt% of soybean protein "Sanraba-10" manufactured by Fuji Oil, Co., Ltd.), 0 to 5 % of GNA was added to obtain specimens. The specimens were subjected to the organoleptic test to evaluate reduction of the soybean smell.

**Table 15**

| | | | | | | |
|---|---|---|---|---|---|---|
| Addition amount of GNA (%) | 0 | 1 | 2 | 3 | 4 | 5 |
| Evaluation results | 3 | 1.1 | 0.6 | 0.2 | 0.1 | 0.1 |

As shown in Table 15, the addition of GNA to the hamburger steak containing the soybean protein of 5 wt% reduced the soybean smell derived from the soybean protein.

### (16) Fish sausage containing soybean protein

To a fish sausage (containing 7.0 wt% of soybean protein "Sanraba-10" manufactured by Fuji Oil, Co., Ltd.), 0 to 3 % of GNA was added to prepare specimens. The specimens were subjected to the organoleptic test to evaluate reduction of the soybean smell.

**Table 16**

| | | | | | | |
|---|---|---|---|---|---|---|
| Addition amount of GNA (%) | 0 | 0.5 | 1.0 | 1.5 | 2.0 | 3.0 |
| Evaluation results | 3 | 2.3 | 2.0 | 1.5 | 1.1 | 0.6 |

As shown in Table 16, the addition of GNA to the hamburger steak containing 7 wt% of the soybean protein reduced the soybean smell derived from the soybean protein.

### (17) Kamaboko containing soybean protein

To Kamaboko (containing 7.0 wt% of soybean protein "Sanraba-10" manufactured by Fuji Oil, Co., Ltd.), 0 to 3 % of GNA was added to prepare specimens. The specimens were subjected to the organoleptic test to evaluate reduction of the soybean smell.

**Table 17**

| | | | | | | |
|---|---|---|---|---|---|---|
| Addition amount of GNA (%) | 0 | 0.5 | 1.0 | 1.5 | 2.0 | 3.0 |
| Evaluation results | 3 | 2.8 | 2.5 | 1.9 | 0.8 | 0.2 |

As shown in Table 17, the addition of GNA to the Kamaboko containing 7 wt% soybean protein reduced the soybean smell derived from the soybean protein.

### Example 6 Reduction of fishy smell

### (18) Fish sauce

To a sample obtained by 20-fold diluting fish sauce ("Ajiro" made in Thailand manufactured by Tosogo Co., Ltd.) with distilled water, 0 to 0.5 % of GNA was added to prepare specimens. The specimens were subjected to the organoleptic test to evaluate reduction of a fishy smell.

**Table 18**

| | | | | | | |
|---|---|---|---|---|---|---|
| Addition amount of GNA (%) | 0 | 0.1 | 0.2 | 0.3 | 0.4 | 0.5 |
| Evaluation results | 3 | 2.7 | 1.1 | 0.6 | 0.3 | 0 |

As shown in Table 18, the addition of GNA to the fish sauce remarkably reduced the fishy smell.

### (19) Dried bonito shavings

20g of dried bonito shavings were added to 600mL of boiling water and then the boiling was immediately stopped to leave to stand. To a supernatant thereof, 0.5 to 1.0 % of GNA was added to prepare specimens. The specimens were subjected to the organoleptic test to evaluate reduction of the fishy smell.

**Table 19**

| | | | | | | |
|---|---|---|---|---|---|---|
| Addition amount of GNA (%) | 0.5 | 0.6 | 0.7 | 0.8 | 0.9 | 1.0 |
| Evaluation results | 2.3 | 2.2 | 1.6 | 0.8 | 0.5 | 0.2 |

As shown in Table 19, the addition of GNA to the solution of the dried bonito extract reduced the fishy smell.

### (20) DHA

A DHA oil was emulsified to prepare emulsion samples each containing 23% DHA. To the samples, 0 to 5 % of GNA was added to obtain specimens, which were subjected to the organoleptic test.

### (Ingredients of the emulsion)

| | |
|---|---|
| DHA oil (containing 46 % DHA) | 50 parts by weight |
| Water | 49 parts by weight |
| Glycerin fatty acid ester | 1 part by weight |

**Table 20**

| | | | | | | |
|---|---|---|---|---|---|---|
| Addition amount of GNA (%) | 0 | 1 | 2 | 3 | 4 | 5 |
| Evaluation results | 3 | 2.8 | 2.6 | 2.5 | 2.5 | 1.7 |

As shown in Table 20, the fishy smell of DHA was reduced by the addition of GNA.

### Example 7 Reduction of vegetable smell

### (21) Vegetable juice

Vegetable juice of 80 parts by weight was obtained from the following vegetables using a juicer and salt of 1.5 % was added thereto to prepare samples. To the samples, 0 to 3 % of GNA was added to obtain specimens. The specimens were subjected to the organoleptic test to evaluate reduction of smells particular to carrot and celery.

### (Ingredients of the juice)

| | |
|---|---|
| Carrot | 40 parts by weight |
| Celery | 10 parts by weight |
| Water | 50 parts by weight |

**Table 21**

| | | | | | |
|---|---|---|---|---|---|
| Addition amount of GNA (%) | 0 | 0.5 | 1.0 | 2.0 | 3.0 |
| Evaluation results | 3 | 2.8 | 2.3 | 2.0 | 1.6 |

As shown in Table 21, the raw smell of the vegetable juice was reduced by the addition of GNA.

### (22) Tomato juice

To commercially available tomato juice (manufactured by KAGOME Co., Ltd.), 0 to 3 % of GNA was added to prepare specimens. The specimens were subjected to the organoleptic test to evaluate reduction of a smell particular to the tomato.

**Table 22**

| | | | | | | | |
|---|---|---|---|---|---|---|---|
| Addition amount of GNA (%) | 0 | 0.5 | 1.0 | 1.5 | 2.0 | 2.5 | 3.0 |
| Evaluation results | 3 | 2.5 | 2.4 | 2.2 | 1.9 | 1.3 | 1.0 |

As shown in Table 22, the addition of GNA to the tomato juice considerably reduced the smell of the tomato.

### (23) Garlic

To 1 part of commercially available grated garlic (manufactured by YOUKI Co., Ltd., ingredients: garlic, salt, alcohol, pH adjuster (vitamin C)), 10 parts of distilled water was added to prepare samples. To the samples, 0 to 3 % of GNA was added to obtain specimens. The specimens were subjected to the organoleptic test to evaluate reduction of a smell of garlic.

**Table 23**

| | | | | | |
|---|---|---|---|---|---|
| Addition amount of GNA (%) | 0 | 0.5 | 1.0 | 2.0 | 3.0 |
| Evaluation results | 3 | 1.5 | 1.3 | 1.2 | 1.0 |

As shown in Table 23, the addition of GNA reduced the garlic smell.

### Example 8 Reduction of smell of old rice

### (24) Old rice

Domestically produced rice (produced in 1996) that had been stored for more than 2 years was washed with water and immersed in water for 30 minutes. After straining water from the rice, it was put in a ceramic rice cocker together with water in a volume of 1.4 times greater than that of the rice and 0 to 2 % of GNA, and cooked in a microwave (500W, 14 minutes, steamed for 15 minutes). The old rice thus cooked in water added with 0 to 2 % of GNA was used as specimens, which were subjected to the organoleptic test to evaluate reduction of a smell of old rice.

**Table 24**

| | | | | | |
|---|---|---|---|---|---|
| Addition amount of GNA (%) | 0 | 0.1 | 0.5 | 1.0 | 2.0 |
| Evaluation results | 3 | 1.0 | 0.4 | 0.3 | 0.3 |

As shown in Table 24, the addition of GNA together with water to the old rice reduced the old rice smell. However, with the addition of 1 % or more of GNA, the smell of GNA itself was perceived.

### Example 9 Reduction of smell of konjak jelly

### (25) Konjak jelly

Konjak jelly noodles, manufactured by TENGU Co., Ltd., were washed with water and then water was drained off them using a colander for 30 minutes. 20 g of the konjak noodles was sealed in a polyethylene bag together with 100 mL of distilled water and heated in water at 85°C for 30 minutes. To the thus treated distilled water, 0 to 5 % of GNA was added to prepare specimens, which were subjected to the organoleptic test to evaluate reduction of a smell of konjak jelly.

**Table 25**

| | | | | | | |
|---|---|---|---|---|---|---|
| Addition amount of GNA (%) | 0 | 0.5 | 1.0 | 2.0 | 3.0 | 5.0 |
| Evaluation results | 3 | 2.1 | 1.9 | 1.6 | 1.2 | 1.0 |

As shown in Table 25, the addition of GNA to the distilled water reduced the smell of the konjak jelly.

### Example 10 Reduction of smell of vitamin

### (26) Vitamin B group

1.8 g of "Lapis Vitamin B group", a dietary supplement containing vitamin B group manufactured by Tokiwa Chemical Industries, Co., Ltd., was grounded and dissolved in distilled water of 100 mL to prepare samples. To the samples, 0 to 3 % of GNA was added to obtain specimens. The specimens were subjected to the organoleptic test to evaluate reduction of a smell particular to the vitamin B group.

**Table 26**

| | | | | |
|---|---|---|---|---|
| Addition amount of GNA (%) | 0 | 1.0 | 3.0 | 5.0 |
| Evaluation results | 3 | 2.1 | 0.8 | 0.5 |

As shown in Table 26, the addition of GNA reduced the smell particular to the vitamin B group.

### Example 11 Reduction of smell of retort-pouched product

### (27) Retort-pouched product

To leek, pork and codfish of 100 g each, 500 mL of distilled water and 0.5 to 3 % of GNA with respect to the distilled water were added, respectively. These were sealed in a retort pack and subjected to retort processing (121°C, 20 minutes), respectively, to obtain specimens. The specimens were subjected to the organoleptic test to evaluate reduction of a smell of the retort-pouched products.

**Table 27**

| Addition amount of GNA (%) | | 0 | 0.5 | 1.0 | 2.0 | 3.0 |
|---|---|---|---|---|---|---|
| Results | Leek | 3 | 2.9 | 2.8 | 1.5 | 1.0 |
| | Pork | 3 | 2.2 | 1.0 | 0.8 | 0.3 |
| | Codfish | 3 | 2.8 | 1.7 | 0.7 | 0.4 |

As shown in Table 27, the addition of GNA to the water in the retort pack (distilled water) reduced the retort smell.

### Example 12 Reduction of smell of animal meat

### (28) Mutton

200 g of frozen mutton was cut into 1cm pieces and mixed with 6 g of table salt. The mixture was subjected to salting at 5°C for 12 hours. To the mixture 40 mL of a solution containing 0 to 10 g of GNA in ice water was added and mixed. The mixture was subjected to cutting and casing and then boiled in water at 70°C for 30 minutes. This was cooled with cold water and kept in cold storage for 12 hours to obtain specimens. The specimens were subjected to the organoleptic test to evaluate reduction of a smell of the mutton.

**Table 28**

| | | | | | | |
|---|---|---|---|---|---|---|
| Addition amount of GNA (%) | 0 | 0.4 | 0.8 | 1.6 | 2.4 | 4.0 |
| Evaluation results | 3 | 2.5 | 1.9 | 1.1 | 0.8 | 0.5 |

As shown in Table 28, the addition of GNA reduced the mutton smell. The specimens obtained in this example were prepared without adding any spices and/or flavoring vegetables. If other ingredients such as spices and the like are used together with the GNA, it is expected that the mutton smell can be reduced with less amount of GNA.

### Example 13 Reduction of smell and improvement of flavor of powdered milk

In this example, an organoleptic test was performed in the same manner as the above except that the effect of masking the smell and improvement of flavor caused by the masking were evaluated under the following evaluation basis.

### Evaluation basis

-2: smell and flavor particular to the powdered milk are considerably intensified
-1: smell and flavor particular to the powdered milk are slightly intensified
0: smell and flavor particular to the powdered milk are unchanged
+ 1: smell and flavor particular to the powdered milk are slightly killed
+2: smell and flavor particular to the powdered milk are considerably killed

### (29) Reconstituted milk

Samples were obtained by mixing 50 g of powdered skim milk ("Skim Milk" manufactured by Snow Brand Milk Products Co., Ltd., containing 34.4 % protein) with 450 mL of water. To the samples, 0 to 0.5 % of GNA was mixed and dissolved and then heated at 75°C for 10 minutes. These were cooled to obtain specimens. The specimens were subjected to the organoleptic test to evaluate reduction of the smell and improvement of the flavor of the powdered skim milk.

**Table 29**

| | | | | | | | |
|---|---|---|---|---|---|---|---|
| Addition amount of GNA (%) | 0 | 0.05 | 0.10 | 0.20 | 0.30 | 0.40 | 0.50 |
| Evaluation results | 0 | +0.4 | +0.7 | +1.0 | +1.5 | +1.6 | +1.8 |

As shown in Table 29, the addition of GNA reduced the smell and improved the flavor, both of which being particular to the powdered skim milk.

### (30) Potage soup

Samples were obtained by mixing 16.5 g of powdered instant soup ("Cup Soup" manufactured by Ajinomoto Co., Inc., containing powdered skim milk "Skim Milk" of Snow Brand Milk Products Co., Ltd.) with 150mL of boiling water. 0 to 1.0% of GNA was mixed to be dissolved into the samples to obtain specimens. The specimens were subjected to the organoleptic test to evaluate reduction of the smell and improvement of the flavor of the powdered skim milk.

**Table 30**

| | | | | | |
|---|---|---|---|---|---|
| Addition amount of GNA (%) | 0 | 0.1 | 0.3 | 0.5 | 1.0 |
| Evaluation results | 0 | +0.8 | + 1.2 | +1.6 | +1.7 |

As shown in Table 30, the addition of GNA reduced the smell and improved the flavor, both of which being particular to the powdered skim milk.

### (31) Cookies

A sample was obtained by mixing 50 g of margarine, 40 g of sugar, 25 g of powdered skim milk ("Skim Milk" of Snow Brand Milk Products Co., Ltd.), 100 g of weak flour, 30 g of egg and a small amount of vanilla extract. The sample was kneaded with 0 to 1.0 % of GNA with respect to the sample and matured at 5°C for 30 minutes. This was rolled out and cut into pieces in a desired shape and then baked at 180°C for 15 minutes to obtain specimens. The specimens were subjected to the organoleptic test to evaluate reduction of the smell and improvement of the flavor of the powdered skim milk.

**Table 31**

| | | | | | |
|---|---|---|---|---|---|
| Addition amount of GNA (%) | 0 | 0.1 | 0.3 | 0.5 | 1.0 |
| Evaluation results | 0 | +0.5 | +0.8 | +1.3 | +1.7 |

As shown in Table 31, the addition of GNA reduced the smell and improved the flavor, both of which being particular to the powdered skim milk.

### (32) Milk jelly

A sample was obtained by mixing 400 mL of reconstituted milk (a solution of 50 g of powdered skim milk "Skim Milk" of Snow Brand Milk Products Co., Ltd. (containing 34.4 % protein) dissolved in 450 mL of water), 10 g of gelatin, 60 g of sugar and a small amount of vanilla extract. 0 to 1.0 % of GNA with respect to the sample was mixed and dissolved under heating. The mixture solution was filled in containers and cooled to obtain specimens. The specimens were subjected to the organoleptic test to evaluate reduction of the smell and improvement of the flavor of the powdered skim milk.

**Table 32**

| | | | | | |
|---|---|---|---|---|---|
| Addition amount of GNA (%) | 0 | 0.1 | 0.3 | 0.5 | 1.0 |
| Evaluation results | 0 | +0.5 | +0.7 | +1.0 | +1.5 |

As shown in Table 32, the addition of GNA reduced the smell and improved the flavor, both of which being particular to the powdered skim milk.

### (33) Milk using WPC

24.6g of WPC "SUNLACT N-5" (containing 34.4 % protein), manufactured by Taiyo Kagaku Co., Ltd., and 475.4mL of water were mixed to prepare a sample. 0 to 1.00 % of GNA with respect to the sample was mixed and dissolved in the sample and heated at 75°C for 10 minutes. The solution was cooled to obtain specimens. The specimens were subjected to the organoleptic test to evaluate reduction of the smell and improvement of the flavor of the WPC.

**Table 33**

| | | | | | | | |
|---|---|---|---|---|---|---|---|
| Addition amount of GNA (%) | 0 | 0.05 | 0.10 | 0.20 | 0.30 | 0.50 | 1.00 |
| Evaluation results | 0 | +0.6 | +0.7 | +0.9 | +1.3 | +1.5 | +1.7 |

As shown in Table 33, the addition of GNA reduced the smell and improved the flavor particular to the WPC.

### Industrial Applicability

The masking agent containing a salt of gluconic acid according to the present invention is remarkably effective in reducing flavor and/or odor particular to an oral ingestible product.

## Claims

1. A masking agent comprising a nontoxic salt of gluconic acid as an active ingredient.

2. A masking agent according to claim 1 for masking odor of an oral ingestible product.

3. A masking agent according to claim 1 for masking flavor of an oral ingestible product.

4. A masking agent according to any one of claims 1 to 3, wherein the nontoxic salt of gluconic acid is an alkali metal salt or an alkali earth metal salt of gluconic acid.

5. A method for masking flavor and/or odor particular to an oral ingestible product by adding a masking agent according to any one of claims 1 to 4.
